# EUROPÄISCHE PATENTSCHRIFT

(11) **EP 0 497 734 B1**
(45) Veröffentlichungstag und Bekanntmachung des Hinweises auf die Patenterteilung: **10.05.2000**
(21) Anmeldenummer: 92810037.9
(22) Anmeldetag: 22.01.1992
(51) Int. Cl.: C07D 251/24

(54) **Verfahren zur Herstellung von 2-(2',4'-Dihydroxyphenyl)-4,6-diaryl-s-triazinen**
Process for the preparation of 2-(2',4'-dihydroxyphenyl)-4,6-diaryl-s-triazines
Procédé pour la préparation des 2-(2',4'-dihydroxyphényl)-4,6-diaryl-s-triazines

(30) Priorität: 31.01.1991 CH 29191; 23.09.1991 CH 278891
(43) Veröffentlichungstag der Anmeldung: 05.08.1992
(73) Patentinhaber: Ciba Specialty Chemicals Holding Inc., 4057 Basel (CH)
(72) Erfinder: Burdeska, Kurt, Dr., CH-4058 Basel (CH)

(56) Entgegenhaltungen:
- EP-A- 0 395 938
- BERICHTE DER DEUTSCHEN CHEMISCHEN GESELLSCHAFT Bd. 23, Nr. 13, 1890, BERLIN, DE Seiten 2919 - 2922; A. PINNER: 'Ueber Diphenyloxykyanidin' *

## Beschreibung

Die vorliegende Erfindung betrifft ein neues, vereinfachtes Verfahren zur Herstellung von 2-(2',4'-Dihydroxyphenyl)-4,6-diaryl-s-triazinen aus 2-Hydroxy-4,6-diaryl-s-triazinen.

Die Umsetzung von 2-Hydroxy-4,6-diaryl-s-triazinen mit Thionylchlorid zu 2-chloro-4,6--diaryl-s-triazinen ist bekannt, z.B. aus der US-A-2,691,018.

Es wurde nun gefunden, dass 2-(2 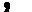 ,4 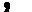 -Dihydroxyphenyl)-4,6-diaryl-s-triazine durch Umsetzung von 2-Hydroxy-4,6-diaryl-s-triazinen mit Thionylchlorid und 1,3-Dihydroxybenzol ohne Isolierung der Chlor-s-triazin-Zwischenstufe in einem einzigen Reaktionsschritt hergestellt werden können.

Gegenstand der vorliegenden Erfindung ist daher ein Verfahren zur Herstellung von 2-(2 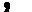 ,4 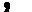 -Dihydroxyphenyl)-4,8-diaryl-s-triazinen der Formel 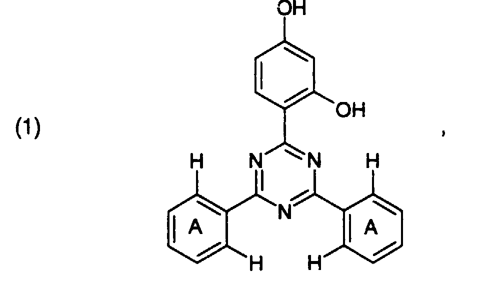 das dadurch gekennzeichnet ist, dass man 2-Hydroxy-4,6-diaryl-s-triazine der Formel 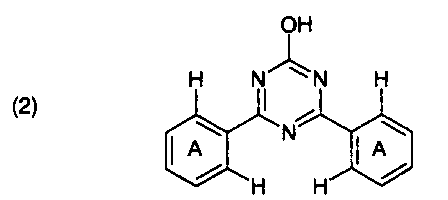 bei einer Temperatur zwischen 100-150°C mit Thionylchlorid in Anwesenheit von katalytischen Mengen eines polaren Lösungsmittels und in Gegenwart eines inerten organischen Lösungsmittels, und anschliessend mit 1,3-Dihydroxybenzol unter Verwendung einer Lewis-Säure in Gegenwart eines inerten organischen Lösungsmittels, umsetzt, wobei in den Formeln (1) und (2) die Ringe A durch C₁-C₅-Alkyl weiter substituiert sein können.

Beispiele für A als C₁-C₅-Alkyl sind Methyl, Ethyl, n-Propyl, Isopropyl, n-Butyl, sek.Butyl, tert.Butyl, Amyl, Isoamyl oder tert.Amyl bzw. Methoxy, Ethoxy, Isopropoxy, Isobutoxy oder tert.Butoxy.

Vorzugsweise kommen Verbindungen der Formeln(1) und (2) in Betracht, bei denen die Ringe A nicht weiter substituiert sind.

Zur Herstellung der Verbindungen der Formel 1 wird als Lewis-Säure vorzugsweise wasserfreies Aluminiumchlorid verwendet.

Die Umsetzung der Verbindung der Formel (2) mit Thionylchlorid und 1,3-Dihydroxybenzol erfolgt im organischen Medium. Es sind dafür verschiedene Lösungsmittel geeignet, die lediglich die eine Bedingung erfüllt haben müssen, unter den gegebenen Reaktionsbedingungen inert zu sein. Als Lösungsmittel kommen beispielsweise in Betracht: aromatische und aliphatische Kohlenwasserstoffe und ihre alkylierten, halogenierten und nitrierten Derivate, wie beispielsweise Nitrobenzol, Chlorbenzol, o-Dichlorbenzol, 1,2,3-Trimethylbenzol, Toluol, Xylol, Xylol-Isomerengemische, Tetrahydronaphthalin, α-Chlornaphthalin, Acetylentetrachlorid, Ethylendichlorid und ähnliche.

Bevorzugt werden für die Umsetzung als Lösungsmittel Toluol, Xylol oder Xylolisomerengemische verwendet.

Neben dem inerten organischen Lösungsmittel verwendet man im ersten Re-aktionsschritt für die Umsetzung mit Thionylchlorid zusätzlich ein polares Lösungsmittel, das einen Siedepunkt aufweist, der zwischen 120 und 150° C liegt. Vorzugsweise wird dafür Dimethylformamid verwendet, das den Vorteil hat, für die Umsetzung nur einen geringen Thionylchlorid-Überschuss von ca. 10 bis 15% einsetzen zu müssen. Verzichtet man auf dieses polare Lösungsmittel, muss man mit einem mehr als dreifachen Thionylchlorid-Überschuss arbeiten, wie in der US-A-2,691,018 beschrieben ist.

Im Vordergrund des Interresses stehen Umsetzungen, bei denen als Lösungsmittel Xylol oder Xylolisomerengemische und Dimethylformamid verwendet werden.

In einer bevorzugten Ausführungsform des erfindungsgemässen Verfahrens wird 2-Hydroxy-4,6-diphenyl-s-triazin mit Thionylchlorid in Anwesenheit von katalytischen Mengen Dimethylformamid und anschliessend mit 1,3-Dihydroxybenzol unter Verwendung von Aluminiumchlorid in Gegenwart von Toluol, Xylol oder Xylolisomerengemischen umgesetzt.

Das erfindungsgemässe Verfahren bietet weiterhin die Möglichkeit, die Reaktion nach der Umsetzung der Ausgangsverbindung der Formel(2) mit Thionylchlorid abzubrechen und ohne grösseren Arbeitsaufwand die Chlor-s-Triazin-Zwischenstufe zu isolieren. Man erhält dann Verbindungen der Formel 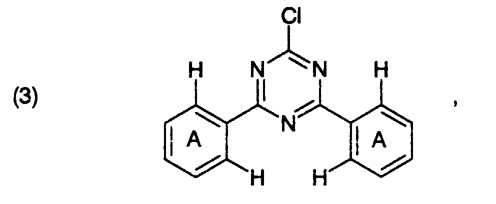 worin A die in den Formeln (1) und (2) angegebene Bedeutung hat, in guten Ausbeuten.

Das Verfahren zur Herstellung von 2-Hydroxy-4,6-diphenyl-s-triazinen der Formel (2) ist dadurch gekennzeichnet, dass man eine wässrige, alkalische Lösung eines Benzamidinhydrohalogenids bei 0 bis 80° C mit einem Halogenkohlensäurealkylester zu einem Benzimidoyl-carbaminsäurealkylester umsetzt, die erhaltene Emulsion mit einem inerten organischen Lösungsmittel versetzt und die entstandene organische Phase enthaltend den gebildeten Benzimidoyl-carbaminsäurealkylester zur Bildung des 2-Hydroxy-4,6-diaryl-s-triazins nach dem Schema 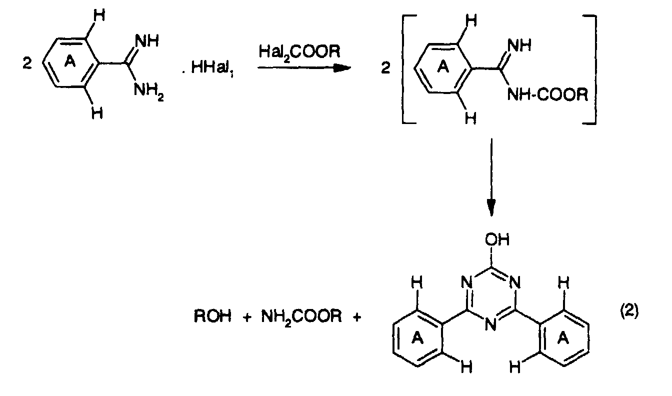 umsetzt,
worin Hal₁ und Hal₂ Halogen und R C₁-C₄Alkyl bedeuten und A die angegebene Bedeutung hat.
Als C₁-C₄Alkyl bedeutet R Methyl, Ethyl, Propyl, Isopropyl, Butyl, sek.Butyl oder tert. Butyl.

Ethyl ist bevorzugt.

Der Ringschluss zum 2-Hydroxy-4,6-diphenyl-s-triazin wird zwecks rascher Entfernung des Ethanols und Urethans auch vorteilhafterweise unter vermindertem Druck zwischen 150 und 200 mbar (150 - 200 hPa) durchgeführt.

Als Benzamidinhydrohalogenid kommen das Benzamidinhydrobromid und vorzugsweise das -chlorid in Betracht.

Bromameisensäureethylester und besonders Chlorameisensäureethylester sind als bevorzugte Halogenkohlensäurealkylester zu nennen.

Die wässrige alkalische Benzamidinhydrohalogenid-Lösung ist vorzugsweise eine Alkalimetallhydroxidlösung, wobei ganz besonders bevorzugt eine Natriumhydroxidlösung verwendet wird.

Die Umsetzung des Benzimidoylcarbaminsäureethylesters zu Verbindungen der Formel(2) erfolgt in einem inerten, organischen Lösungsmittel mit einem Siedepunkt von 160 bis 250° C. Als Lösungsmittel kommen dafür beispielsweise in Betracht: o-Dichlorbenzol, Nitrobenzol, 1,2,3-Trimethylbenzol, ein Gemisch aus 26,5% Diphenyl und 73,5% Diphenylether (®Dowtherm) oder Anisol. Als bevorzugte Lösungsmittel sind o-Dichlorbenzol und 1,2,3-Trimethylbenzol bzw. ®Dowtherm zu nennen.

Die Reaktion zu Verbindungen der Formel (2) wird gegebenenfalls unter Phasentransferbedingungen und Zuhilfenahme eines Phasentransferkatalysators wie Tetrabutylammoniumbromid, Butyltriethylammoniumbromid oder -chlorid, Butyltributylammoniumbromid oder -chlorid durchgeführt.
Die Ausgangsverbindungen entsprechend Formel (2) sind bekannt, z.B. aus A. Pinner, Chem. Ber. 23, 1919 (1890).

Gegegenüber dem dort beschriebenen Verfahren werden die Verbindungen der Formel (2) nach dem erfindungsgemässen Verfahren ohne Isolierung des Benzimidoyl-carbaminsäurealkylesters in einem einstufigen Verfahren erhalten. Ausserdem wird das Hydroxyphenyl-s-triazin entsprechend Formel (2) in so guter Reinheit erhalten, dass es mit Vorteil ohne weitere Isolierung zu einem 2-(2',4'-Dihydroxyphenyl)-4.6-diaryl-s-triazin der Formel (1) weiterverarbeitet werden kann. Diese Verbindungen lassen sich also auf einfache Weise und in guten Ausbeiten in einem Eintopfverfahren herstellen.

Gegenstand der Erfindung ist somit auch ein Verfahren zur Herstellung 2-(2',4'-Dihydroxyphenyl)-4,6-diphenyl-s-triazinen der Formel (1), das dadurch gekennzeichnet, dass man eine wässrige, alkalische Lösung eines Benzamidinhydrohalogenids bei 0 bis 80° C mit einem Halogenkohlensäurealkylester zu einem Benzimidoyl-carbaminsäurealkylester umsetzt, die erhaltene Emulsion mit einem inerten organischen Lösungsmittel versetzt und die entstandene organische Phase enthaltend den gebildeten Benzimidoyl-carbaminsäurealkylester zu 2-Hydroxy-4,6-diphenyl-s-triazin der Formel 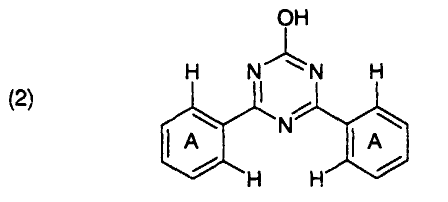 worin A die unter Formel 1 angegebene Bedeutung hat, umsetzt, und die Verbindung der Formel (2) bei einer Temperatur zwischen 100 und 150° C mit Thionylchlorid in Anwesenheit von katalytischen Mengen eines polaren Lösungsmittels und in Gegenwart eines inerten organischen Lösungsmittels, und anschliessend mit 1,3-Dihydroxybenzol unter Verwendung einer Lewis-Säure in Gegenwart eines inerten organischen Lösungsmittels umsetzt.

Ein besonders bevorzugtes Verfahren zur Herstellung von 2-(2',4'-Dihydroxyphenyl)-4,6-diphenyl-s-triazinen gemäss Formel (1) ist dadurch gekennzeichnet, dass man ein Benzamidinhydrohalogenid mit einem Halogenkohlensäurealkylester unter vermindertem Druck von 150-200 hPa (150-200 mbar) zu 2-Hydroxy-4,6-diphenyl-s-triazin der Formel (2) umsetzt und dieses Zwischenprodukt ohne Isolierung bei einer Temperatur zwischen 100-150° mit Thionylchlorid in Anwesenheit von katalytischen Mengen eines polaren Lösungsmittels und in Gegenwart eines inerten organischen Lösungsmittels und anschliessend mit 1,3-Dihydroxybenzol unter Verwendung einer Lewis-Säure in Gegenwart eines inerten organischen Lösungsmittels zur Verbindung der Formel (1) umsetzt.

Mit dem vorliegenden Verfahren lassen sich 2-(2',4'-Dihydroxyphenyl)-4,6-diphenyl-s-triazine und auch die Ausgangsverbindungen auf einfache Weise und in guten Ausbeuten herstellen.

Die nach dem erfindungsgemässen Verfahren hergestellten Verbindungen finden Verwendung als UV-Absorber oder sind Ausgangsprodukte für die Herstellung von UV-Absorbern.

Aus der EP-A-0 395 938 ist ein Verfahren zur Herstellung von 2-(2 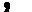 ,4 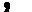 -Dihydroxyphenyl)-4,6-diphenyl-s-triazinen bekannt, welches sich in charakteristischer Weise dadurch von dem erfindungegemässen Verfahren unterscheidet, dass als Ausgangsverbindung der Formel (2) eine Verbindung mit einer -SCH₃ Gruppe an Stelle der -OH Gruppe eingesetzt wird.

Die folgenden Beispiele veranschaulichen die Erfindung. Die Temperaturen sind in °C angegeben.

### Herstellung der Ausgangsverbindungen

Beispiel 1: 47 g Benzamidinhydrochlorid werden in 250 ml Wasser gelöst und mit 80 g einer 30%-igen Natriumhydroxidlösung versetzt. Unter schnellem Rühren und leichter Kühlung lässt man danach zum Reaktionsgemisch in ca. 5 Minuten 32,6 g Chlorameisensäureethylester zufliessen, wobei die Temperatur auf 40-45° ansteigt. Zur Beendigung der Reaktion wird noch 14 Stunden bei 45-50° gerührt. Anschliessend lässt man zur entstandenen Emulsion 150 ml o-Dichlorbenzol zufliessen. Nach kurzem Nachrühren wird die o-Dichlorbenzolphase von der wässrigen Phase abgetrennt, in einem Kolben mit Destillationsaufsatz zum Sieden erhitzt und 14 Stunden bei Siedetemperatur gehalten (175-180°). Nach dem Abkühlen auf Raumtemperatur wird das ausgefallene Produkt abfiltriert, zweimal mit Methanol gewaschen und bei 80° im Vakuum getrocknet. Das Produkt wird zur Reinigung in 250 ml einer Lösung von Methanol/Wasser im Verhältnis 8:2 für 15 Minuten zum Sieden erhitzt und dann durch Filtration isoliert. Man erhält eine Verbindung der Formel

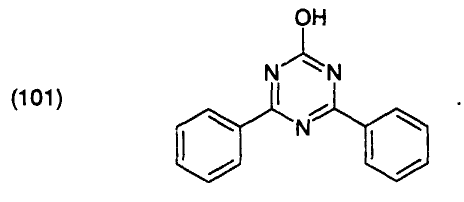

Die Ausbeute des erhaltenen Rohproduktes beträgt nach dem Trocknen 30,5 g (= 81,6% der Theorie). Der Schmelzpunkt liegt nach einer Umkristallisation bei 296-298°.

Beispiel 2: 412,2 g einer 38%-igen methanolischen Benzamidinhydrochloridlösung werden mit 500 ml 1,2,3-Trimethylbenzol vermischt. Unter schwachem Vakuum werden bei 40-50° 300 ml Methanol abdestilliert. Nach Zugabe von 100 ml Wasser und 80 g einer 50%igen wässrigen Natriumhydroxidlösung wird das Gemisch für weitere 30 Minuten bei 20-30° gerührt. Anschliessend werden dem Reaktionsgemisch 150 ml Wasser, 80 g einer 50%-igen Natriumhydroxidlösung und 1 g Tetrabutylammoniumbromid zugesetzt. Unter schnellem Rühren (500 U/min) werden nun bei 15-20° unter Kühlung in einer Stunde 112,9 g Chlorameisensäureethylester zugetropft. Der pH-Wert wird dabei durch Zugabe von 50%iger Natronlauge bei 8-8,5 gehalten. Es wird noch eine Stunde nachgerührt, danach auf 70° erhitzt und das Wasser von der organischen Phase abgetrennt. Diese wird, wie in Beispiel 1 beschrieben, unter gleichzeitiger Abdestillation von Ethanol für zwei Stunden auf 170-175° erhitzt. Nach dem Abkühlen und Reinigung mit Methanol/Wasser erhält man 105 g (= 84,3% der Theorie) des Rohproduktes der Verbindung der Formel(101) mit einem Schmelzpunkt von 295-297° (nach Umkristallisation).

Beispiel 3(a): 157,6 g Benzamidinhydrochlorid (Reinheit: 99,4%) werden mit 250 ml Wasser und 80 g einer 50%-igen Natriumhydroxidlösung 30 Minuten bei Raumtemperatur gerührt. Nach Zugabe von weiteren 80 g Natronlauge werden unter schnellem Rühren (500 U/min) in einer Stunde bei 15-20° unter Kühlung 112,9 g Chlorameisensäureethylester zugetropft. Es resultiert ein Kristallbrei, der noch eine Stunde bei Raumtemperatur gerührt wird. Nach Zusatz von 100 ml 1,2,3-Trimethylbenzol wird auf 85 erhitzt, wobei das kristalline Produkt in Lösung geht. Nach Abtrennung der wässrigen Phase lässt man die organische Phase in einer Stunde in 400 ml 1,2,3-Trimethylbenzol bei 170-175° unter gleichzeitiger Abdestillation des entstandenen Alkohols zufliessen. Es wird noch eine Stunde bei 170-175° nachgerührt, dann auf Raumtemperatur abgekühlt und das ausgefallene Produkt abfiltriert. Nach Reinigung, wie in Beispiel 1 angegeben, erhält man 105,5 g der Verbindung der Formel (101). Der Schmelzpunkt liegt zwischen 296 und 297° (nach Umkristallisation).

Beispiel 4: 157,6 g Benzamidinhydrochlorid (Reinheit: 99,4%) werden mit 300 ml Wasser, 112,9 g Chlorameisensäureethylester und 160 g einer 50%-igen Natriumhydroxidlösung wie in Beispiel 3(a) angegeben, umgesetzt. Zur entstandenen Suspension lässt man 300 ml des Lösungsmittels ®Dowtherm zufliessen. Nach Erwärmung auf 70° und Abtrennung der wässrigen Phase wird die Lösungsmittelphase (®Dowtherm) unter Vakuum von 200 mbar auf 170° erhitzt, wobei Ethanol und Urethan abdestillieren. Nach Beendigung der Reaktion wird das ausgefallene Produkt auf 70° abgekühlt, mit 200 ml Methanol versetzt und danach bei Raumtemperatur abfiltriert. Es wird mit Methanol gewaschen und bei 120 im Vakuum getrocknet. Die Ausbeute beträgt 108 g (86% der Theorie). Die Kontrolle durch Dünnschichtchromatographie zeigt ein reines Produkt, das nicht umkristallisiert werden muss.

### Herstellung der Chlor-s-triazin-Zwischenstufe

Beispiel 5: 24,9 g 2-Hydroxy-4,6-diphenyl-s-triazin werden in 175 ml Xylolisomerengemisch auf 80° erhitzt. Nach Zugabe von 2 ml Dimethylformamid lässt man unter gutem Rühren innerhalb von 30 Minuten 13,1 g Thionylchlorid zufliessen. Danach wird 30 Minuten bei 85-90° und anschliessend noch 3 Stunden bei 125-130° gerührt. Die entstandene Lösung wird zur Trockne eingedampft. Man erhält 26 g (= 97,1% der Theorie) der Verbindung der Formel 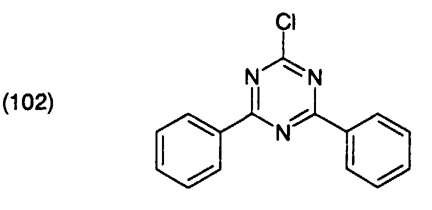 mit dem Schmelzpunkt 139-140°.

### Herstellung der Endprodukte:

Beispiel 6: 124,6 g 2-Hydroxy-4,6-diphenyl-1,3,5-s-triazin werden in 600 ml Xylol-Isomerengemisch suspendiert und nach Zusatz von 3 ml Dimethylformamid auf 100° erhitzt. Unter gutem Rühren lässt man danach bei 100-105° innerhalb von einer Stunde 71,4g Thionylchlorid zufliessen. Die dünne Suspension wird noch eine Stunde bei gleicher Temperatur gerührt und danach bis zur vollständigen Lösung auf 125-130° erhitzt. Nach dem Abkühlen auf 100° werden unter leichtem Vakuum 100 ml Xylol-Isomerengemisch abdestilliert. Die Lösung wird auf 60° abgekühlt und innerhalb von einer Stunde mit 73,3g wasserfreiem Aluminiumchlorid (Fa. Merck) versetzt. Es wird nun erneut auf 80° erhitzt und unter gutem Rühren eine Suspension von 66,1g 1,3-Dihydroxybenzol in 100ml Xylolisomerengemisch in 30 Minuten zudosiert. Zur Vervollständigung der Reaktion wird noch für weitere 5 Stunden bei 85° gerührt. Das Reaktionsgemisch wird danach in ca. 15 Minuten mit einem Gemisch aus 450 ml Wasser und 50 ml einer 30%igen Salzsäure versetzt, anschliessend auf 100° erhitzt und das Xylolisomerengemisch mit Wasserdampf abdestilliert. Das fest angefallene Produkt wird bei 80° filtriert, mit heissem Wasser säurefrei gewaschen und bei 80-90° im Vakuum getrocknet. Die Reinigung erfolgt durch Behandlung mit einem Gemisch aus Dimethylformamid/Wasser im Verhältnis 7:3 bei 90°. Man erhält 151,8 g (= 88% der Theorie) der Verbindung der Formel 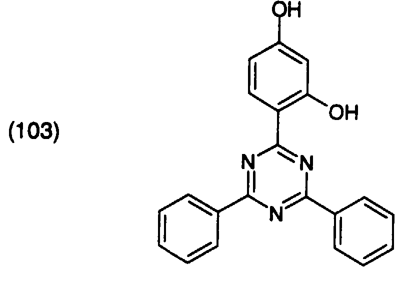 vom Schmelzpunkt 272-273°.

## Patentansprüche

1. Verfahren zur Herstellung von 2-(2',4'-Dihydroxyphenyl)-4,6-diphenyl-s-triazinen der Formel 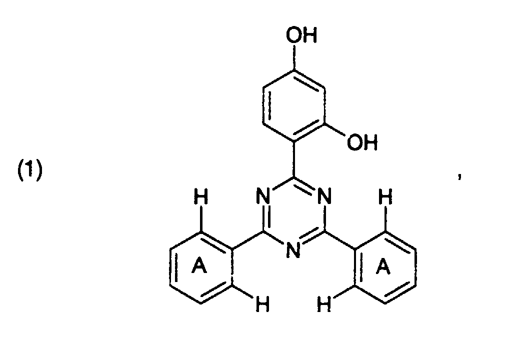 dadurch gekennzeichnet, dass man 2-Hydroxy-4,6-diphenyl-s-triazine der Formel 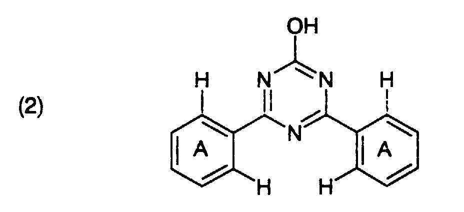 bei einer Temperatur zwischen 100-150° C mit Thionylchlorid in Anwesenheit von katalytischen Mengen eines polaren Lösungsmittels und in Gegenwart eines inerten organischen Lösungsmittels, und anschliessend mit 1,3-Dihydroxybenzol unter Verwendung einer Lewis-Säure in Gegenwart eines inerten organischen Lösungsmittels, umsetzt, wobei in den Formeln (1) und (2) die Ringe A durch C₁-C₅-Alkyl weiter substituiert sein können.

2. Verfahren nach Anspruch 1, dadurch gekennzeichnet, dass die Ringe A der Formeln (1) und (2) nicht weiter substituiert sind.

3. Verfahren gemäss einem der Ansprüche 1 oder 2, dadurch gekennzeichnet, dass man als Lewis-Säure wasserfreies Aluminiumchlorid verwendet.

4. Verfahren gemäss einem der Ansprüche 1 bis 3, dadurch gekennzeichnet, dass als inertes organisches Lösungsmittel Toluol, Xylol oder Xylolisomerengemische verwendet werden.

5. Verfahren gemäss einem der Ansprüche 1 bis 4, dadurch gekennzeichnet, dass man als polares Lösungsmittel Dimethylformamid verwendet.

6. Verfahren gemäss einem der Ansprüche 1 bis 5, dadurch gekennzeichnet, dass man als Lösungsmittel Xylol oder Xylolisomerengemische und Dimethylformamid verwendet.

7. Verfahren gemäss einem der Ansprüche 1 bis 6, dadurch gekennzeichnet, dass man 2-Hydroxy-4,6-diphenyl-s-triazin mit Thionylchlorid in Anwesenheit von katalytischen Mengen Dimethylformamid und anschliessend mit 1,3-Dihydroxybenzol unter Verwendung von wasserfreiem Aluminiumchlorid in Gegenwart von Toluol, Xylol oder Xylolisomerengemischen umsetzt.

8. Verfahren zur Herstellung von 2-(2',4'-Dihydroxyphenyl)-4,6-diphenyl-s-triazinen gemäss Anspruch 1, dadurch gekennzeichnet, dass man eine wässrige, alkalische Lösung eines Benzamidinhydrohalogenids bei 0 bis 80° C mit einem Halogenkohlensäurealkylester zu einem Benzimidoyl-carbaminsäurealkylester umsetzt, die erhaltene Emulsion mit einem inerten organischen Lösungsmittel versetzt und die entstandene organische Phase enthaltend den gebildeten Benzimidoyl-carbaminsäurealkylester zu 2-Hydroxy-4,6-diphenyl-s-triazin der Formel 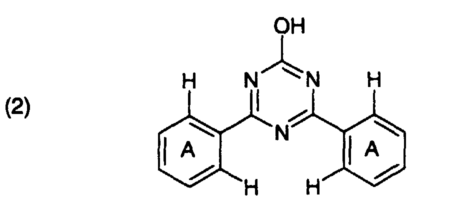 worin A die in Anspruch 1 angegebene Bedeutung hat, umsetzt, und die Verbindung der Formel (2) bei einer Temperatur zwischen 100 und 150° C mit Thionylchlorid in Anwesenheit von katalytischen Mengen eines polaren Lösungsmittels und in Gegenwart eines inerten organischen Lösungsmittels, und anschliessend mit 1,3-Dihydroxybenzol unter Verwendung einer Lewis-Säure in Gegenwart eines inerten organischen Lösungsmittels umsetzt.

9. Verfahren gemäss Anspruch 8, dadurch gekennzeichnet, dass die Umsetzung des Benzimidoyl-carbaminsäurealkylester zu 2-Hydroxy-4,6-diphenyl-s-triazin der Formel (2) unter vermindertem Druck von 150-200 hPa (150-200 mbar) durchgeführt wird.

10. Verfahren gemäss Anspruch 8 oder 9, dadurch gekennzeichnet, dass als Benzamidinhydrohalogenid Benzamidinhydrochlorid verwendet wird.

11. Verfahren gemäss einem der Ansprüche 8 bis 10, dadurch gekennzeichnet, dass als Halogenkohlensäureester Chlor- oder Bromameisensäureethylester verwendet wird.

12. Verfahren gemäss einem der Ansprüche 8 bis 11, dadurch gekennzeichnet, dass als alkalische Lösung des Benzamidinhydrohalogenids eine Natriumhydroxidlösung verwendet wird.

13. Verfahren gemäss einem der Ansprüche 8 bis 12, dadurch gekennzeichnet, dass die erhaltene, Benzimidoyl-carbaminsäurealkylester enthaltende Emulsion mit einem inerten organischen Lösungsmittel mit einem Siedepunkt von 160 bis 250° C versetzt wird.

14. Verfahren gemäss Anspruch 13, dadurch gekennzeichnet, dass als inertes organisches Lösungsmittel o-Dichlorbenzol, Nitrobenzol, 1,2,3-Trimethylbenzol, ein Gemisch aus 26,5% Diphenyl und 73,5% Diphenylether oder Anisol verwendet wird.

15. Verfahren zur Herstellung von 2-(2',4'-Dihydroxyphenyl)-4,6-diphenyl-s-triazinen gemäss Anspruch 1, dadurch gekennzeichnet, dass man ein Benzamidinhydrohalogenid mit einem Halogenkohlensäurealkylester unter vermindertem Druck von 150-200 hPa (150-200 mbar) zu 2-Hydroxy-4,6-diphenyl-s-triazin der Formel (2) umsetzt und dieses Zwischenprodukt ohne Isolierung bei einer Temperatur zwischen 100-150° mit Thionylchlorid in Anwesenheit von katalytischen Mengen eines polaren Lösungsmittels und in Gegenwart eines inerten organischen Lösungsmittels und anschliessend mit 1,3-Dihydroxybenzol unter Verwendung einer Lewis-Säure in Gegenwart eines inerten organischen Lösungsmittels zur Verbindung der Formel (1) umsetzt.

## Claims

1. A process for the preparation of a 2-(2 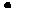 ,4 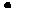 -dihydroxyphenyl)-4,6-diphenyl-s-triazine of formula 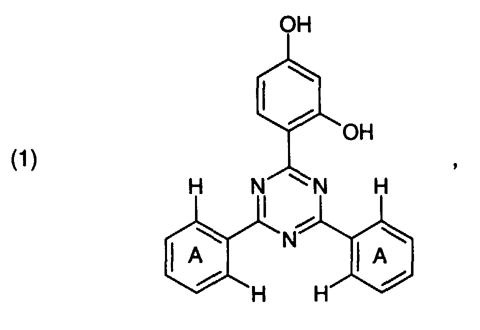 which comprises reacting a 2-hydroxy-4,6-diphenyl-s-triazine of formula 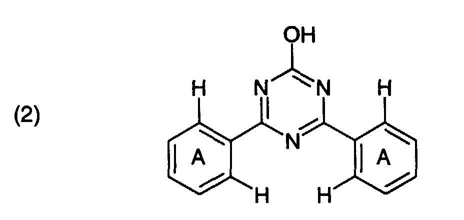 in the temperature range from 100-150° C with thionyl chloride in the presence of catalytic amounts of a polar solvent and in the presence of an inert organic solvent and subsequent reaction with 1,3-dihydroxybenzene using a Lewis acid in the presence of an inert organic solvent, in which formulae (1) and (2) the rings A may be further substituted by C₁-C₅alkyl.

2. A process according to claim 1, wherein the rings A of formulae (1) and (2) are not further substituted.

3. A process according to either claim 1 or claim 2, wherein the Lewis acid is anhydrous aluminium chloride.

4. A process according to any one of claims 1 to 3, wherein the inert organic solvent is selected from the group consisting of toluene, xylene and xylene isomer mixtures.

5. A process according to any one of claims 1 to 4, wherein the polar solvent is dimethyl formamide.

6. A process according to any one of claims 1 to 5, wherein the solvent is selected from the group consisting of xylene and mixtures of xylene isomers and dimethyl formamide.

7. A process according to any one of claims 1 to 6, which comprises reacting 2-hydroxy-4,6-diphenyl-s-triazine with thionyl chloride in the presence of catalytic amounts of dimethyl formamide and subsequent reaction with 1,3-dihydroxybenzene using anhydrous aluminium chloride in the presence of toluene, xylene or xylene isomer mixtures.

8. A process for the preparation of a 2-(2 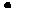 ,4 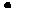 -dihydroxyphenyl)-4,6-diphenyl-s-triazine according to claim 1, which comprises reacting an aqueous alkaline solution of a benzamidine hydrohalide in the temperature range from 0 to 80° C with an alkyl halocarbonate to an alkyl benzimidoylcarbamate, charging the emulsion so obtained with an inert organic solvent and reacting the resulting organic phase containing the alkyl benzimidoylcarbamate to give the 2-hydroxy-4,6-diphenyl-s-triazine of formula 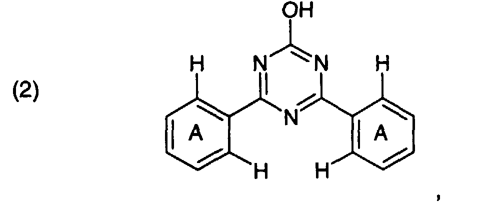 wherein A has the meaning defined in claim 1, and reacting the compound of formula (2) in the temperature range from 100 to 150° C with thionyl chloride in the presence of catalytic amounts of a polar solvent and in the presence of an inert organic solvent, and subsequent reaction with 1,3-dihydroxybenzene using a Lewis acid in the presence of an inert organic solvent.

9. A process according to claim 8, which comprises reacting the alkyl benzimidoylcarbamate to the 2-hydroxy-4,6-diphenyl-s-triazine of formula (2) under a reduced pressure of 150-200 hPa (150-200 mbar).

10. A process according to either claim 8 or claim 9, wherein the benzamidine hydrohalide is benzamidine hydrochloride.

11. A process according to any one of claims 8 to 10, wherein the halocarbonate is ethyl chlorocarbonate or ethyl bromocarbonate.

12. A process according to any one of claims 8 to 11, wherein the alkaline solution of the benzamidine hydrohalide is a sodium hydroxide solution.

13. A process according to any one of claims 8 to 12, which comprises charging the alkyl benzimidoylcarbamate-containing emulsion obtained with an inert organic solvent having a melting point of 160 to 250° C.

14. A process according to claim 13, wherein the inert organic solvent is o-dichlorobenzene, nitrobenzene, 1,2,3-trimethylbenzene, a mixture of 26.5% of diphenyl and 73.5% of diphenyl ether, or anisole.

15. A process for the preparation of a 2-(2',4'-dihydroxyphenyl)-4,6-diphenyl-s-triazine according to claim 1, which comprises reacting a benzamidine hydrohalide with an alkyl halocarbonate under a reduced pressure of 150-200 hPa (150-200 mbar) to the 2-hydroxy-4,6-diphenyl-s-triazine of formula (2) and reacting this intermediate without isolation in the temperature range from 100-150°C with thionyl chloride in the presence of catalytic amounts of a polar solvent and in the presence of an inert organic solvent and subsequent reaction with 1,3-dihydroxybenzene using a Lewis acid in the presence of an inert organic solvent to the compound of formula (1).

## Revendications

1. Procédé de préparation de 2-(2',4'-dihydroxyphényl)-4,6-diphényl-s-triazines de formule 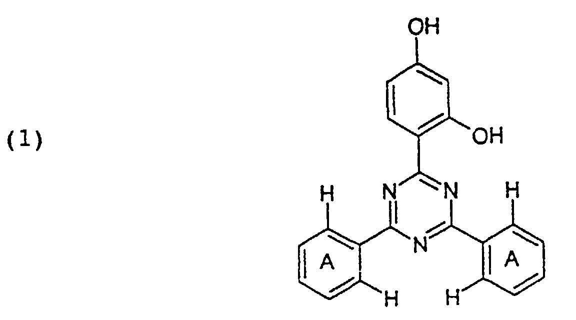 caractérisé en ce que l'on fait réagir des 2-hydroxy-4,6-diphényl-s-triazines de formule 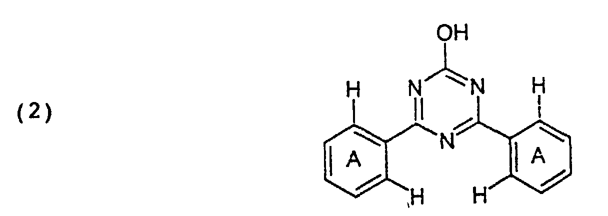 avec le chlorure de thionyle à une température comprise entre 100 et 150°C en présence de quantités catalytiques d'un solvant polaire et en présence d'un solvant organique inerte, et ensuite avec le 1,3-dihydroxybenzène, en utilisant un acide de Lewis en présence d'un solvant organique inerte, les noyaux A dans les formules (1) et (2) pouvant être encore substitués par des groupes alkyle en C₁ à C₅.

2. Procédé selon la revendication 1, caractérisé en ce que les noyaux A des formules (1) et (2) ne sont pas plus substitués.

3. Procédé selon l'une des revendications 1 ou 2, caractérisé en ce que l'on utilise comme acide de Lewis le chlorure d'aluminium anhydre.

4. Procédé selon l'une des revendications 1 à 3, caractérisé en ce que l'on utilise comme solvant organique inerte le toluène, le xylène ou des mélanges d'isomères de xylène.

5. Procédé selon l'une des revendications 1 à 4, caractérisé en ce que l'on utilise comme solvant polaire le diméthylformamide.

6. Procédé selon l'une des revendications 1 à 5, caractérisé en ce que l'on utilise comme solvants le xylène ou des mélanges d'isomères de xylène et le diméthylformamide.

7. Procédé selon l'une des revendications 1 à 6, caractérisé en ce que l'on fait réagir la 2-hydroxy-4,6-diphényl-s-triazine avec le chlorure de thionyle en présence de quantités catalytiques de diméthylformamide, et ensuite avec le 1,3-diméthylbenzène en utilisant du chlorure d'aluminium anhydre en présence de toluène, de xylène ou de mélanges d'isomères de xylène.

8. Procédé de préparation de 2-(2',4'-dihydroxyphényl)-4,6-diphényl-s-triazines selon la revendication 1, caractérisé en ce que l'on fait réagir une solution alcaline aqueuse d'un hydrohalogénure de benzamidine à une température de 0 à 80°C avec un halogénocarboxylate d'alkyle pour former un benzimidoylcarbamate d'alkyle, l'on ajoute à l'émulsion obtenue un solvant organique inerte et l'on fait réagir la phase organique résultante contenant le benzimidoylcarbamate d'alkyle formé pour obtenir la 2-hydroxy-4,6-diphényl-s-triazine de formule 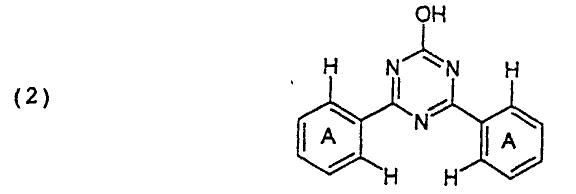 dans laquelle A a la signification indiquée dans la revendication 1 et en ce que le composé de formule (2) réagit avec du chlorure de thionyle à une température comprise entre 100 et 150°C en présence de quantités catalytiques d'un solvant polaire et en présence d'un solvant organique inerte et ensuite avec le 1,3-dihydroxybenzène en utilisant un acide de Lewis en présence d'un solvant organique inerte.

9. Procédé selon la revendication 8, caractérisé en ce que la réaction du benzimidoylcarbamate d'alkyle pour donner la 2-hydroxy-4,6-diphényl-s-triazine de formule (2) s'effectue sous une pression réduite de 150 à 200 hPa (150 à 200 mbar).

10. Procédé selon la revendication 8 ou 9, caractérisé en ce que l'hydrohalogénure de benzamidine utilisé est l'hydrochlorure de benzamidine.

11. Procédé selon l'une des revendications 8 à 10, caractérisé en ce que les halogénocarboxylates utilisés sont le chloro- ou le bromoformiate d'éthyle.

12. Procédé selon l'une des revendications 8 à 11, caractérisé en ce que la solution alcaline de l'hydrohalogénure de benzamidine utilisée est une solution d'hydroxyde de sodium.

13. Procédé selon l'une des revendications 8 à 12, caractérisé en ce que l'émulsion obtenue contenant du benzimidoylcarbamate d'alkyle additionnée d'un solvant organique inerte dont le point d'ébullition est de 160 à 250°C.

14. Procédé selon la revendication 13, caractérisé en ce que le solvant organique inerte utilisé est l'o-dichlorobenzène, le nitrobenzène, le 1,2,3-triméthylbenzène, un mélange de 26,5% de diphényle et 73,5% de diphényléther ou l'anisol.

15. Procédé pour la préparation de 2-(2',4'-dihydroxyphényl)-4,6-diphényl-s-triazines selon la revendication 1, caractérisé en ce que l'on fait réagir un hydrohalogénure de benzamidine avec un halogénocarboxylate d'alkyle sous une pression réduite de 150 à 200 hPa (150 à 200 mbar) pour obtenir la 2-hydroxy-4,6-diphényl-s-triazine de formule (2), et l'on fait réagir ce produit intermédiaire, sans être séparé, avec du chlorure de thionyle à une température comprise entre 100 et 150° en présence de quantités catalytiques d'un solvant polaire et en présence d'un solvant organique inerte et ensuite avec du 1,3-dihydroxybenzène en utilisant un acide de Lewis en présence d'un solvant organique inerte pour obtenir le composé de formule (1).
